# EUROPÄISCHE PATENTSCHRIFT

(11) **EP 0 531 875 B1**
(45) Veröffentlichungstag und Bekanntmachung des Hinweises auf die Patenterteilung: **24.03.2004**
(21) Anmeldenummer: 92114982.9
(22) Anmeldetag: 02.09.1992
(51) Int. Cl.: C07D 501/34, A61K 31/545, C07D 501/00

(54) **Diastereomer des 3-Cephem-4-carbonsäure-1-(-isopropoxycarbonyloxy)ethylesters und Verfahren zu dessen Herstellung**
Diastereomer of the 3-cephem-4-carboxylic acid-1-(-isopropoxycarbonyloxy)ethylester and process for its preparation
Diastéréomère de l'ester 1-(1-isopropoxycarbonyloxy)éthyle de l'acide 3-céphen-4-carboxylique et procédé pour sa préparation

(30) Priorität: 07.09.1991 DE 4129771
(43) Veröffentlichungstag der Anmeldung: 17.03.1993
(73) Patentinhaber: Aventis Pharma Deutschland GmbH, 65929 Frankfurt am Main (DE)
(72) Erfinder: Fischer, Gerd, Dr., W-6250 Limburg 8 (DE); Defossa, Elisabeth, Dr., W-6270 Idstein/Ts. (DE); Gerlach, Uwe, Dr., W-6230 Frankfurt/M. 80 (DE); Hörlein, Rolf, Dr., W-6000 Frankfurt/M. 71 (DE); Krass, Norbert, Dr., W-6000 Frankfurt/M. (DE); Lattrell, Rudolf, Dr., W-6240 Königstein/Ts. (DE); Stache, Ulrich, Dr., W-6238 Hofheim/Ts. (DE); Wollmann, Theodor, Dr., W-6238 Hofheim/Ts. (DE); Isert, Dieter, Dr., W-6236 Eschborn (DE)

(56) Entgegenhaltungen:
- EP-A- 0 034 536
- EP-A- 0 049 118
- EP-A- 0 379 132
- GB-A- 2 110 688
- CHEMICAL ABSTRACTS, vol. 102, no. 25, 24. Juni 1985, Columbus, Ohio, US; abstract no. 220658k, Seite 572 ;Spalte R ;

## Beschreibung

Gegenstand der Erfindung ist das verbessert enteral resorbierbare polarere Diastereomer des (6R,7R)-7-[2-(2-Aminothiazol-4-yl)-2(Z)-(methoxyimino)-acetamido]-3-(methoxymethyl)-3-cephem-4-carbonsäure-1-(isopropoxycarbonyloxy)-ethylesters der Formel I und dessen physiologisch unbedenkliche Salze 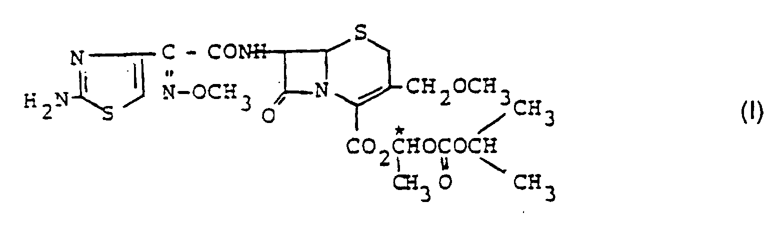 sowie Verfahren zu ihrer Herstellung.

In dem U.S. Patent Nr. 4,486,425 werden Ester der (6R,7R)-7-[2-(2-Aminothiazol-4-yl)-2(Z)-(methoxyimino)acetamido]-3-(methoxymethyl)-3-cephem-4-carbonsäure beschrieben. Von diesen ist der Ester der Formel I von besonderem Interesse, da er in verschiedenen Tierspezies und am Menschen gut enteral resorbiert wird und nach der Resorption durch körpereigene Enzyme wieder rasch und vollständig zum antibiotisch aktiven Cephalosporin mit freier Carboxylgruppe gespalten wird. Diese Verbindung ist unter dem Namen Cefpodoxim Proxetil (Drugs of the Future 14, 73 (1989)) bekannt.

Der Ester der Formel I besitzt zwei jeweils (R)-konfigurierte asymmetrische Kohlenstoffatome in der 6- und 7-Stellung des Cephemgerüstes sowie ein asymmetrisches Kohlenstoffatom in der 1-Stellung der Ethyloxyestergruppe -O-CH(CH₃)-O-. Die in U.S. 4,486,425 beschriebenen Verbindungen liegen als Mischungen der Diastereomeren bezüglich des asymmetrischen Kohlenstoffatoms der 1-Ethyloxyestergruppe -O-CH(CH₃)-O- vor. Vergleichbare Mischungen von Diastereomeren liegen z.B. auch beim Cefotiam-Hexetil (Drugs of the Future 13, 230 (1988)), Cefuroxim-Axetil (Drugs of the Future 10, 112 (1985)), Baccefuzonam (N.A. Kuck et al., Proc. 14^{th} Int. Congr. Chemother. 2, 1137 (1985)) und BMY28271 (The Journal of Antibiotics 43, 1564 (1990)) vor.

Nach den bisherigen Versuchen zum Mechanismus der enteralen Resorption derartiger Cephem-Prodrugester hat die Konfiguration in der 1-Position der Ethylestergruppe -O-CH(CH₃)-O- keinen Einfluß auf die Höhe der enteralen Resorbierbarkeit. Dies konnte beispielweise für die Diastereomeren von Cefotiam-Hexetil experimentell gezeigt werden (T.Nishimura et al., The Journal of Antibiotics 40, 81-90 (1987)).

Im Falle von Cefotiam-Hexetil wurden die beiden Diastereomeren chromatographisch getrennt. Dieser Weg ist jedoch verlustreich und nicht allgemein gangbar, da die physikalischen Eigenschaften der beiden Diastereomeren, wie beispielsweise beim Cefpodoxim Proxetil, zu ähnlich sind um eine chromatographische Trennung zu ermöglichen. Außerdem sind beide Diastereomere bzw. das Diastereomerengemisch unter den Bedingungen der Säulenchromatographie zersetzlich.

Die beiden getrennten Diastereomeren von Cefpodoxim Proxetil sind deshalb bisher nicht beschrieben worden. Es sind auch keine präparativen Verfahren bekannt geworden, um gezielt die beiden Diastereomeren von Cephalosporinesterprodrugs herzustellen, die wie Cefpodoxim Proxetil von dem 1-Ethyloxyesterrest -O-CH(CH₃)-O- abgeleitet sind.

Daher war es überraschend, daß die getrennten Diastereomeren der Formel I deutliche Unterschiede bei der enteralen Resorption zeigen, so daß das besser resorbierbare polare Diastereomere eine höhere Bioverfügbarkeit als das Diastereomerengemisch Cefpodoxim Proxetil zeigte.

Gegenstand der vorliegenden Erfindung ist daher die polarere diastereomerenreine Verbindungen der Formel I, in der die Gruppe =N-OCH₃ in der syn-Position steht. Das polarere der beiden Diastereomeren besitzt die höhere Bioverfügbarkeit.

Gegenstand der vorliegenden Erfindung ist ferner ein diastereomerenreines Salz der allgemeinen Formel II 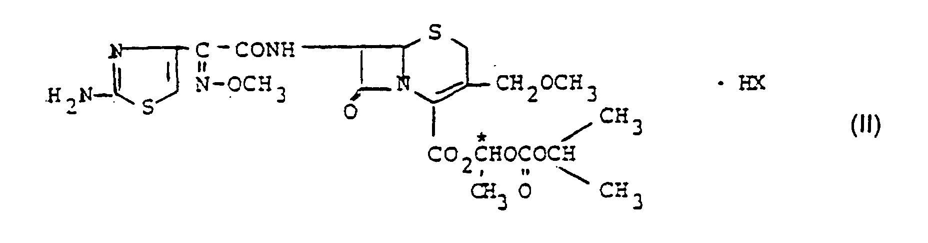 wobei die Verbindung der Formel (I) das polarere Diastereomer ist und wobei HX für eine ein- oder mehrbasische Säure steht und wobei X ein anorganisches oder organisches physiologisch unbedenkliches Anion sein kann.

Als anorganische Säure bedeutet HX beispielsweise die stöchiometrische Menge an HCl, HBr, HJ, HBF₄, HNO₃, HClO₄, H₂SO₄ oder H₃PO₄. Als organische Säure steht HX für aliphatische oder aromatische Sulfonsäuren. Die anorganischen Säuren HCl, HBr und H₂SO₄ sowie die organischen Säuren Methansulfonsäure, Ethansulfonsäure, Benzolsulfonsäure, p-Toluolsulfonsäure und 4-Ethylbenzolsulfonsäure sind besonders bevorzugt.

Gegenstand dieser Erfindung ist weiterhin ein Verfahren zur Herstellung von diastereomerenreinen Verbindungen der Formel I, das dadurch gekennzeichnet ist, daß eine Zwischenstufe der Formel III 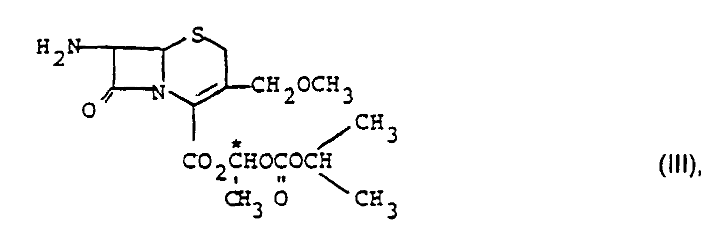 oder der Formel IV 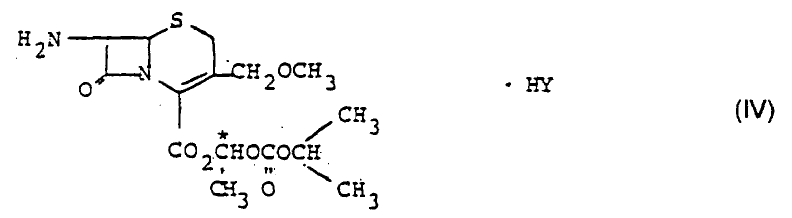 in diastereomerenreiner Form hergestellt und in das reine Diastereomer der Formel I oder der Formel II überführt wird.

Das so erhaltene reine Diastereomer der Formel I wird in die Salze der Formel 11 nach an sich bekannten Methoden überführt, wie sie beispielsweise für analoge Verbindungen beschrieben wurden.

Die Verbindung der Formel III oder deren Salze der allgemeinen Formel IV stellt man nach an sich bekannten Verfahren, die beispielsweise in der Patentanmeldung JP 60,004,190A beschrieben wurden, als Mischung der Diastereomeren her.

Die Diastereomeren können durch fraktionierte Kristallisation von Salzen der Formel IV getrennt werden. In der allgemeinen Formel IV steht HY für eine ein- oder mehrbasische Säure, wobei Y ein anorganisches oder organisches Anion sein kann. HClO₄, HSCN, H₂SO₄ oder H₃PO₄. Als organische Säure steht HY für aliphatische bzw. aromatische Sulfonsäuren, Carbonsäuren oder Phosphonsäuren. So können beispielsweise die folgenden organischen Säuren eingesetzt werden: Benzolsulfonsäure, p-Toluolsulfonsäure, 4-Ethylbenzolsulfonsäure, 4-Chlorbenzolsulfonsäure, 4-Brombenzolsulfonsäure, 2-Mesitylensulfonsäure, 4-Biphenylsulfonsäure, Naphtalin-1,5-disulfonsäure, Methansulfonsäure, Ethansulfonsäure, Dodecylsulfonsäure, Camphersulfonsäure, Oxalsäure.

Als bevorzugte Säurekomponenten müssen angesehen werden: HCl, HBr, Benzolsulfonsäure, p-Toluolsulfonsäure, 4-Ethylbenzolsulfonsäure und 4-Biphenylsulfonsäure.

Die Herstellung des Salzes der Formel IV erfolgt durch Zusammengeben einer Lösung der Diastereomerenmischung der Formel III und einer Lösung der Säurekomponente HY. Als organische Lösungsmittel können z.B. Ester, Ether, Alkohole, Ketone, Nitrile, chlorierte Kohlenwasserstoffe und Kohlenwasserstoffe sowie deren Mischungen eingesetzt werden. Bevorzugte Lösungsmittel sind z.B. Benzol, Toluol, Ethylacetat, Butylacetat, Methanol, Ethanol, n-Propanol, iso-Propanol, tert. Butanol, Diisopropylether, Aceton, Acetonitril und Dichlormethan und deren Mischungen.

Als Lösungsmittel für anorganische Säuren kann zudem Wasser eingesetzt werden, wenn das organische Lösungsmittel mit Wasser mischbar ist. Lösungen von HCl und HBr in organischen Lösungsmitteln können beispielsweise durch Einleiten von Chlorwasserstoff- oder Bromwasserstoffgas erzeugt werden. Lösungen von HCl und HBr in organischen Lösungsmitteln können auch aus Acetylhalogeniden, Phosphorhalogeniden und Phosphoroxyhalogeniden und einem Alkohol erzeugt werden (Halogen = Cl, Br).

Wichtig für die Trennung der Diastereomeren ist das Verhältnis der Base der Formel III zur Säurekomponente. Für ein Äquivalent der Diastereomerenmischung der Formel III sollten 0.2-2.0, bevorzugt 0.4-1.5 Äquivalente Säurekomponente eingesetzt werden.

Für das Verfahren ist es wesentlich, daß die Fällung der reinen Diastereomeren der allgemeinen Formel IV in zwei aufeinanderfolgenden Teilschritten erfolgt. So wird beispielsweise durch Zusammengeben einer Lösung des Diastereomerengemisches der Formel III mit einer Lösung der Säurekomponente HY zunächst das schwerer lösliche Diastereomere der allgemeinen Formel IV gefällt, durch Filtration abgetrennt, und anschließend aus der Filtrationslösung das leichter lösliche Diastereomere der allgemeinen Formel IV gefällt. Bei den aufeinanderfolgenden Teilschritten kann die Säurekomponente HY gleich oder verschieden sein, wobei die Reihenfolge der Zugabe unterschiedlicher Säurekomponenten HY beliebig ist. So kann beispielsweise durch geeignete Wahl der Säurekomponente HY zunächst das polarere Diastereomere der allgemeinen Formel IV oder das unpolarere Diastereomere der allgemeinen Formel IV als schwerer lösliches Salz gefällt werden.

Durch die Wahl der Säurekomponente können somit beide Diastereomere der Formel IV in reiner Form erhalten werden. So erhält man z.B. bei der Verwendung von Chlorwasserstoff oder Bromwasserstoff zunächst bevorzugt das polarere Diastereomere, während die Verwendung von Benzolsulfonsäure, 4-Ethylbenzolsulfonsäure, Biphenylsulfonsäure oder p-Toluolsulfonsäure bevorzugt das weniger polare Diastereomere liefert.

Die nach Filtration erhaltenen Salze werden, falls notwendig, durch Kristallisation weiter gereinigt. Dazu werden die oben beschriebenen Lösungsmittel und deren Mischungen eingesetzt. Die Auswahl des optimalen Lösungsmittels hängt von der verwendeten Säurekomponente ab. So sind z.B. für das p-Toluolsulfonsäuresalz und für das Hydrochlorid Methanol, Ethanol, n-Propanol, iso-Propanol, Acetonitril, Essigester und Dichlormethan besonders geeignet.

Die Zugabe der Säurekomponente erfolgt bei etwa -10°C bis +50°C, bevorzugt bei +10°C bis +30°C. Abhängig von der Säurekomponente und dem Lösungsmittel rührt man zur Vervollständigung der Fällung noch bis zu etwa 10 Stunden nach. Gegebenenfalls muß zur Vervollständigung der Fällung auf Temperaturen zwischen Raumtemperatur und etwa -78°C gekühlt werden.

Alternativ kann man Diastereomerenmischungen der Formel III auch ausgehend von Verbindungen der Formel V 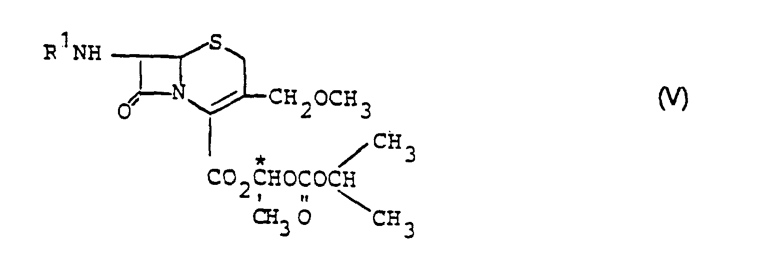 erhalten.

Die Gruppe R¹ stellt dabei eine in der Peptidchemie übliche Aminoschutzgruppe, wie z.B. die Formylgruppe, die tert. Butoxycarbonylgruppe, die Chloracetylgruppe, die Phenoxyacetylgruppe, die Phenylacetylgruppe, die Allyloxcarbonylgruppe, die Benzyloxycarbonylgruppe und die 4-Nitrobenzyloxycarbonylgruppe dar.

Die Abspaltung der Schutzgruppen erfolgt nach an sich bekannten Methoden. So kann die Formylgruppe und die tert. Butoxycarbonylgruppe beispielsweise mit Säure abgespalten werden. Die Phenoxyacetylgruppe und die Phenylacetylgruppe kann beispielsweise mit Phosphorpentachlorid oder enzymatisch mit Penicillin--Acylasen abgespalten werden. Bei der Allyloxycarbonylgruppe kann die Abspaltung mit Pd[P(C₆H₅)₃] erfolgen. Die Benzyloxycarbonylgruppe und die 4-Nitrobenzyloxycarbonylgruppe können hydrogenolytisch entfernt werden.

Bei der Abspaltung der Phenoxyacetylgruppe oder der Phenylacetylgruppe mit Phosphorpentachlorid erhält man das polarere Diastereomere als Hydrochlorid auch ohne Chlorwasserstoffzusatz. Als Quelle für den Chlorwasserstoff dienen bei der Aufarbeitung nicht entfernte Phosphorsäureesterchloride, die langsam Chlorwasserstoff freisetzen.

Ausgehend von Verbindungen der Formel V kann man zu diastereomerenreinen Verbindungen der Formel III oder der allgemeinen Formel IV kommen, indem man zunächst die Trennung der Diastereomeren durchführt, die Schutzgruppe abspaltet und gegebenenfalls die Diastereomerenmischung der allgemeinen Formel IV mit einem Überschuss der Säurekomponente HY fällt. Die Trennung der Diastereomeren der Formel V kann durch Kristallisation oder Chromatographie erfolgen, wobei die genauen Bedingungen von der Schutzgruppe R¹ abhängen. Steht z.B. R¹ für die Phenoxyacetylgruppe, so können die Diastereomeren durch Chromatographie an Kieselgel mit einem organischen Lösungsmittelgemisch getrennt werden.

Ausgehend von den diastereomerenreinen Salzen der Formel IV stellt man nach an sich bekannten Methoden die diastereomerenreinen Basen der Formel III her und überführt diese, wie beispielsweise in der Patentanmeldung JP60,004,189A für die Diastereomerenmischung beschrieben, in das reine Diastereomer der Formel I.

Dazu können die diastereomerenreinen Verbindungen der Formel III beispielsweise mit einer Verbindung der allgemeinen Formel VI 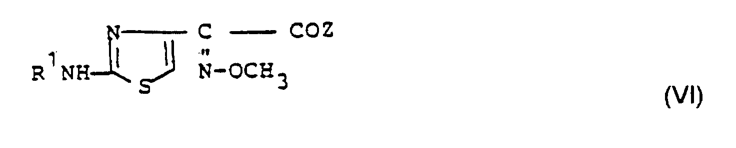 umgesetzt werden, wobei R¹ für Wasserstoff steht oder die oben für Verbindungen der Formel V beschriebene Bedeutung besitzt, und Z für eine in der beta--Lactamchemie übliche aktivierende Gruppe, wie z.B. Chlorid, p-Toluolsulfonyl, 1-Benzotriazolyloxy oder Mercaptobenzothiazolyl steht.

Die unvorhersehbaren, vorteilhaften Eigenschaften der vorliegenden Erfindung liegen in einer erhöhten enteralen Resorption (entsprechend der Wiederfindungsrate) für das polarere Diastereomere der Formel I, wie es in Tabelle 1 gezeigt wird.

**Tabelle 1:**

| | |
|---|---|
| Diastereomerenzusammensetzung | Wiederfindungsrate |
| Diastereomer A (Vergleichsbeispiel 7) | 25 % |
| Diastereomer B (Beispiel 8) | 45 % |

Tabelle 1 zeigt die Wiederfindungsrate (0 - 24 h) von (6R,7R)-7-[2-(2-Aminothiazol-4-yl)-2-(Z)-(methoxyimino)-acetamido]-3-(methoxymet hyl)-3-cephem-4-carbonsäure im Urin von Hunden nach oraler Gabe der diastereomeren Prodrugester (Dosis: 10 mg/kg bezogen auf den biologisch aktiven Wirkstoff).

Die erfindungsgemäßen Verbindungen der allgemeinen Formel I werden in Form von üblichen pharmazeutischen Zubereitungen, wie z.B. Kapseln, Tabletten, Pulvern, Sirupen oder Suspensionen oral verabreicht. Die Dosis hängt vom Alter, den Symptomen und dem Körpergewicht des Patienten sowie von der Dauer der Behandlung ab. Sie liegt jedoch in der Regel zwischen etwa 0.2 g und etwa 5 g täglich, vorzugsweise zwischen etwa 0.5 g und etwa 3 g täglich. Die Verbindungen werden vorzugsweise in aufgeteilten Dosen verabreicht, beispielsweise 2 bis 4mal täglich, wobei die Einzeldosis beispielsweise zwischen 50 und 500 mg Wirkstoff enthalten kann.

Die oralen Zubereitungen können die üblichen Trägerstoffe und/oder Verdünnungsmittel enthalten. So kommen beispielsweise für Kapseln oder Tabletten Bindemittel, wie z.B. Gelatine, Sorbitol, Polyvinylpyrrolidon oder Carboxymethylcellulose, Verdünnungsmittel, wie z.B. Lactose, Zucker, Stärke, Calciumphosphate oder Polyethylenglykol, Gleitstoffe, wie z.B. Talkum oder Magnesiumstearat in Betracht. Für flüssige Zubereitungen, z.B. wäßrige oder ölige Suspensionen, sind Sirupe oder ähnliche bekannte Zubereitungsformen geeignet.

Die folgenden Ausführungsbeispiele für erfindungsgemäß herstellbare, diastereomerenreine Verbindungen der Formel I und Formel II dienen zur weiteren Erläuterung der Erfindung, schränken sie jedoch nicht darauf ein.

### Experimenteller Teil

### Beispiel 1

### (6R,7R)-7-Amino-3-methoxymethyl-3-cephem-4-carbonsäure-1-(isopropoxycarbonyloxy)ethylester-p-toluolsulfonat (Diastereomerengemisch)

1.22 g (5 mmol) (6R,7R)-7-Amino-3-methoxymethyl-3-cephem-4-carbonsäure wurde unter Argonatmosphäre in 15 ml Dichlormethan suspendiert und durch Zusatz von 0.75 ml (5 mmol) DBU in Lösung gebracht. Bei 0°C setzte man 1.43 g (5.5 mmol) 1-Jodethyl-isopropylcarbonat (The Journal of Antibiotics 40, 370 (1987)) zu, rührte noch 40 Minuten bei 0°C und 30 Minuten bei 20°C und verdünnte zur Aufarbeitung mit 50 ml Ethylacetat. Man wusch mit ges. wäßr. NaHCO₃ und NaCl-Lösung, trocknete mit MgSO₄ und engte die organische Phase i.Vak. ein. Das Rohprodukt nahm man in 5 ml Ethylacetat auf und setzte bei 20°C eine Lösung von 1.0 g (5.3 mmol) p-Toluolsulfonsäure-Monohydrat in 5 ml Ethylacetat zu. Man setzte noch 10 ml Diisopropylether zu, kühlte auf 0°C und saugte das ausgefallene Produkt ab.
Ausbeute: 1.93 g (71% d.Th.).
¹H-NMR (DMSO-d₆, 270 MHz): d = 1.25 (m, 6H, C(CH₃)₂); 1.50 (d, 3H, CH-CH₃); 2.30 (s, 3H, Aryl-CH₃); 3.23 (s, 3H, CH₂OCH₃); 3.70 (2H, m, S-CH₂); 4.21 (m, CH₂OCH₃); 4.81 (m, 1H, O-CH(CH₃)₂); 5.25 (m, 2H, H-6 und H-7); 6.81 und 6.85 (2xq, 1 H, O-CH(CH₃)-O); 7.11 und 7.48 (2xd, 4H, Aryl-H) 9.05 (br s, 2H, NH₂). DC (Toluol/Essigester 1 + 1): R_{f} = 0.34 (Diastereomer A) und 0.26 (Diastereomer B).
HPLC: C18 Nukleosil 7 µm; Wasser (+0.1% NH₄OAc) + (Methanol-Wasser 80:20 (+0.1% NH₄OAc)) 45:55, 1 ml/min = 10.8 Min. (Diastereomer A), 9.1 Min. (Diastereomer B).

### Beispiel 2

### (6R,7R)-7-Amino-3-methoxymethyl-3-cephem-4-carbonsäure-1-(isopropoxycarbonyloxy)ethylester (Diastereomerengemisch)

2.53 g (4.6 mmol) Diastereomerengemisch aus Beispiel 1 wurden in einer Mischung von Ethylacetat und 5%iger wäßr. NaHCO₃-Lösung aufgenommen und 5 Min. verrührt. Man trennte die Phasen, wusch die organische Phase mit ges. wäßr. NaCl-Lösung, trocknete mit MgSO₄ und engte i.Vak. ein.
Ausbeute: 1.74 g (100 % d.Th.).

### Beispiel 3

### I) (6R,7R)-7-Amino-3-methoxymethyl-3-cephem-4-carbonsäure-1-(isopropoxycarbonyloxy)ethylester-p-toluolsulfonat (Diastereomer A)

1.74 g (4.63 mmol) Diastereomerengemisch aus Beispiel 2 wurden in 4 ml Ethylacetat aufgenommen und eine Lösung von 0.44 g (2.32 mmol) p-Toluolsulfonsäure-Monohydrat in 3 ml Ethylacetat zugesetzt. Man setzte noch 3 ml Diisopropylether zu und saugte das ausgefallene Produkt ab. Die Filtrationslösung wurde wie unter Beispiel 3 (II) beschrieben weiterverwendet.
Ausbeute: 0.904 g (36 % d.Th.) Diastereomer A (p-Toluolsulfonat).
¹H-NMR (DMSO-d₆, 270 MHz): d = 1.25 (m, 6H, C(CH₃)₂); 1.50 (d, 3H, CH-CH₃); 2.30 (s, 3H, Aryl-CH₃); 3.23 (s, 3H, CH₂OCH₃); 3.69 (2H, ABq, S-CH₂); 4.21 (m, CH₂OCH₃); 4.79 (m, 1 H, O-CH(CH₃)₂); 5.25 (m, 2H, H-6 und H-7); 6.81 (q, 1 H, O-CH(CH₃)-O); 7.11 und 7.48 (2xd, 4H, Aryl-H); 8.9 (br s, 2H, NH₂).
DC (Toluol/Essigester 1 + 1): R_{f} = 0.34.
HPLC: C18 Nukleosil 7 µm; Wasser (+0.1% NH₄OAc) + (Methanol-Wasser 80:20 (+0.1% NH₄OAc)) 45:55, 1 ml/min = 10.8 Min. (Diastereomer A).

### II) (6R,7R)-7-Amino-3-methoxymethyl-3-cephem-4-carbonsäure-1-(isopropoxycarbonyloxy)ethylester-p-toluolsulfonat (Diastereomer B)

Die aus Beispiel 3 (I) erhaltene Filtrationslösung wurde mit einer Lösung von 0.44 g (2.32 mmol) p-Toluolsulfonsäure-Monohydrat in 3 ml Ethylacetat versetzt und das ausgefallene Produkt abgesaugt.
Ausbeute: 0.534 g (21 % d.Th.) Diastereomer B (p-Toluolsulfonat).
¹H-NMR (DMSO-d₆, 270 MHz): d = 1.25 (m, 6H, C(CH₃)₂); 1.50 (d, 3H, CH-CH₃); 2.30 (s, 3H, Aryl-CH₃); 3.23 (s, 3H, CH₂OCH₃); 3.69 (2H, m, S-CH₂); 4.21 (m, CH₂OCH₃); 4.79 (m, 1 H, O-CH(CH₃)₂); 5.25 (m, 2H, H-6 und H-7); 6.84 (q, 1 H, O-CH(CH₃)-O); 7.11 und 7.48 (2xd, 4H, Aryl-H); 8.9 (br s, 2H, NH₂).
DC (Toluol/Essigester 1 + 1): R_{f} = 0.26.
HPLC: C18 Nukleosil 7 µm; Wasser (+0.1% NH₄OAc) + (Methanol-Wasser 80:20 (+0.1% NH₄OAc)) 45:55, 1 ml/min = 9.1 Min. (Diastereomer B).

### Beispiel 4

### I) (6R,7R)-7-Amino-3-methoxymethyl-3-cephem-4-carbonsäure-1-(isopropoxycarbonyloxy)ethylester-hydrochlorid (DiastereomerB)

1.71 g (4.57 mmol) Diastereomerengemisch aus Beispiel 2 wurden in 4 ml Ethylacetat aufgenommen und 0.914 ml (2.28 mmol) 2.45M isopropanolische Salzsäure zugesetzt. Der entstandene Niederschlag wurde abgesaugt und die Filtrationslösung wie unter Beispiel 4 (II) beschrieben weiterverwendet.
Ausbeute: 0.628 g (41 % d.Th.) Diastereomer B (Hydrochlorid).
¹H-NMR (DMSO-d₆, 270 MHz): d = 1.25 (m, 6H, C(CH₃)₂); 1.48 (d, 3H, CH-CH₃); 3.23 (s, 3H, CH₂OCH₃); 3.68 (2H, m, S-CH₂); 4.21 (s, CH₂OCH₃); 4.81 (m, 1H, O-CH(CH₃)₂); 5.21 (q, 2H, H-6 und H-7); 6.85 (q, 1H, O-CH(CH₃)-O); 9.2 (br s, 2H, NH₂).
DC (Toluol/Essigester 1+1): R_{f} = 0.26.
HPLC: C18 Nukleosil 7 µm; Wasser (+0.1% NH₄OAc) + (Methanol-Wasser 80:20 (+01% NH₄OAc)) 45:55, 1 ml/min = 9.1 Min. (Diastereomer B).

### II) (6R,7R)-7-Amino-3-methoxymethyl-3-cephem-4-carbonsäure-1-(isopropoxycarbonyloxy)ethylester-p-toluolsulfonat (Diastereomer A)

Die aus Beispiel 4 (I) erhaltene Filtrationslösung wurde mit einer Lösung von 0.573 g (3.0 mmol) p-Toluolsulfonsäure-Monohydrat in 3 ml Ethylacetat versetzt und das ausgefallene Produkt abgesaugt.
Ausbeute: 0.808 g (38 % d.Th.) Diastereomer A (p-Toluolsulfonat), identisch mit dem Produkt aus Beispiel 3 (I).

### Beispiel 5

### (6R,7R)-7-Amino-3-methoxymethyl-3-cephem-4-carbonsäure-1-(isopropoxycarbonyloxy)ethylester (Diastereomer A)

4.83 g (8.8 mmol) Diastereomer A aus Beispiel 4 (II) wurden in einer Mischung von 80 ml Ethylacetat und 153 ml Wasser mit 0.96 g (11.45 mmol) NaHCO₃ aufgenommen und 5 Min. verrührt. Man trennte die Phasen, wusch die organische Phase mit ges. wäßr. NaCl-Lösung, trocknete mit MgSO₄ und engte i.Vak. ein.
Ausbeute: 3.29 g (100 % d.Th.).

### Beispiel 6

### (6R,7R)-7-Amino-3-methoxymethyl-3-cephem-4-carbonsäure-1-(isopropoxycarbonyloxy)ethylester (Diastereomer B)

4.99 g (12.0 mmol) Diastereomer B aus Beispiel 4 (I) wurden in einer Mischung von 110 ml Ethylacetat und 219 ml Wasser mit 1.36 g (16.28 mmol) NaHCO₃ aufgenommen und 5 Min. verrührt. Man trennte die Phasen, wusch die organische Phase mit ges. wäßr. NaCl-Lösung, trocknete mit MgSO₄ und engte i.Vak. ein.
Ausbeute: 4.49 g (100 % d.Th.).

### Beispiel 7

### (6R,7R)-7-[2-(2-Aminothiazol-4-yl)-2(Z)-(methoxyimino)acetamido]--3-methoxymethyl-3-cephem-4-carbonsäure-1-(isopropoxycarbonyloxy)ethylester (Diastereomer A)

3.29 g (8.8 mmol) Diastereomer A aus Beispiel 5 wurden in 22ml trockenem Dichlormethan unter Argonatmosphäre gelöst und 3.19 g (9.11 mmol) 2-(2-Aminothiazol-4-yl)-2(Z)-(methoxyimino)mercaptobenzothiazolylacetat zugegeben. Man rührte die Suspension noch 1 Stunde bei 20°C, verdünnte dann mit 200 ml Ethylacetat, extrahierte zweimal mit Wasser, trocknete mit MgSO₄ und zog das Lösungsmittel i.Vak. ab. Den Rückstand reinigte man durch Säulenchromatographie (SiO₂, Toluol/Ethylacetat).
Ausbeute: 1.1 g (22 % d.Th.) Diastereomer A.
¹H-NMR (DMSO-d₆, 270 MHz): d = 1.23 (dd, 6H, C(CH₃)₂); 1.49 (d, 3H, CH-CH₃); 3.21 (s, 3H, CH₂OCH₃); 3.48 (2H, ABq, S-CH₂); 3.83 (s, 3H, N-OCH₃); 4.14 (s, CH₂OCH₃); 4.80 (m, 1 H, O-CH(CH₃)₂); 5.21 (d, 1H, H-6); 5.82 (dd, 1 H, H-7); 6.72 (s, 1 H, Thiazol-H); 6.80 (q, 1 H, O-CH(CH₃)-O); 7.2 (br s, 2H, NH₂); 9.59 (d, 1 H, CONH).
HPLC: C18 Nucleosil, 7 µm; Wasser + 1.2-Dimethoxyethan (+EDTA 10 mg/l, +0.2% N-Methylmorpholin, +HClO₄, pH 3.34) 68:32; 1.5 ml/min; 12.6 Min. (Diastereomer A).

### Beispiel 8

### (6R,7R)-7-[2-(2-Aminothiazol-4-yl)-2(Z)-(methoxyimino)acetamido]--3-methoxymethyl-3-cephem-4-carbonsäure-1-(isopropoxycarbonyloxy)ethylester (Diastereomer B)

4.49 g (12.0 mmol) Diastereomer B aus Beispiel 6 wurden in 30ml trockenem Dichlormethan unter Argonatmosphäre gelöst und 4.39 g (12.42 mmol) 2-(2-Aminothiazol-4-yl)-2(Z)-(methoxyimino)mercaptobenzothiazolylacetat zugegeben. Man rührte die Suspension noch 1 Stunde bei 20°C, verdünnte dann mit 200 ml Ethylacetat, extrahierte zweimal mit Wasser, trocknete mit MgSO₄ und zog das Lösungsmittel i.Vak. ab. Den Rückstand reinigte man durch Säulenchromatographie (SiO₂, Toluol/Ethylacetat).
Ausbeute: 4.6 g (69 % d.Th.) Diastereomer B.
¹H-NMR (DMSO-d₆, 270 MHz): d = 1.25 (dd, 6H, C(CH₃)₂); 1.50 (d, 3H, CH-CH₃); 3.21 (s, 3H, CH₂OCH₃); 3.53 (2H, ABq, S-CH₂); 3.85 (s, 3H, N-OCH₃); 4.14 (s, CH₂OCH₃); 4.81 (m, 1H, O-CH(CH₃)₂); 5.19 (d, 1H, H-6); 5.81 (dd, 1H, H-7); 6.72 (s, 1 H, Thiazol-H); 6.83 (q, 1H, O-CH(CH₃)-O); 7.2 (br s, 2H, NH₂); 9.59 (d, 1 H, CONH).
HPLC: C18 Nucleosil, 7 µm; Wasser + 1.2-Dimethoxyethan (+EDTA 10 mg/l, +0.2% N-Methylmorpholin, HClO₄, pH 3.34) 68:32; 1.5 ml/min; 9.7 Min. (Diastereomer B).

### Beispiel 9

### (6R,7R)-7-[2-(2-Aminothiazol-4-yl)-2(Z)-(methoxyimino)acetamido]--3-methoxymethyl-3-cephem-4-carbonsäure-1-(isopropoxycarbonyloxy)ethylester (Diastereomerengemisch, Cefpodoxim Proxetil)

4.26 g (17.5 mmol) (6R,7R)-7-Amino-3-methoxymethyl-3-cephem-4-carbonsäure wurde unter Argonatmosphäre in 40 ml Dichlormethan suspendiert und durch Zusatz von 2.65 g (17.5 mmol) DBU in Lösung gebracht. Bei 0°C setzte man 4.96 g (19.2 mmol) 1-Jodethyl-isopropylcarbonat (The Journal of Antibiotics 40, 370 (1987)) zu, rührte noch 60 Minuten bei 0°C und 20 Minuten bei 20°C. Dann wurden 6.4 g (18.3 mmol) 2-(2-Aminothiazol-4-yl)-2(Z)-(methoxyimino)mercaptobenzothiazolylacetat zugegeben. Man rührte die Suspension noch 2 Stunden bei 20°C, verdünnte dann mit 200 ml Ethylacetat, extrahierte zweimal mit Wasser, trocknete mit MgSO₄ und zog das Lösungsmittel i.Vak. ab. Den Rückstand reinigte man durch Säulenchromatographie (SiO₂, Toluol/Ethylacetat).
Ausbeute: 3.42 g (35 % d.Th.) Diastereomerengemisch A+B.
¹H-NMR (DMSO-d₆, 270 MHz): d = 1.25 (m, 6H, C(CH₃)₂); 1.49 (m, 3H, CH-CH₃); 3.21 (s, 3H, CH₂OCH₃); 3.54 (2H, ABq, S-CH₂); 3.85 (s, 3H, N-OCH₃); 4.14 (s, CH₂OCH₃); 4.8 (m, 1H, O-CH(CH₃)₂); 5.21 (m, 1H, H-6); 5.82 (m, 1 H, H-7); 6.72 (s, 1 H, Thiazol-H); 6.80 und 6.83 (2xq, 1 H, O-CH(CH₃)-O); 7.2 (br s, 2H, NH₂); 9.6 (m, 1H, CONH).
HPLC: C18 Nucleosil, 7 µm; Wasser + 1.2-Dimethoxyethan (+EDTA 10 mg/l, +0.2% N-Methylmorpholin, +HClO₄, pH 3.34) 68:32; 1.5 ml/min; 12.6 Min. (Diastereomer A), 9.7 Min. (Diastereomer B).

## Patentansprüche (Patentansprüche für folgende(n) Vertragsstaat(en): AT, BE, CH, DE, DK, FR, GB, IE, IT, LI, LU, NL, PT, SE)

1. Diastereomere der Verbindung (6R,7R)-7-[2-(2-Aminothiazol-4-yl)-2-(Z)-(methoxyimino)acetamido]-3-(methoxymethyl)-3-cephem-4-carbonsäure-1-(isopropoxycarbonyloxy)ethylesters der Formel I 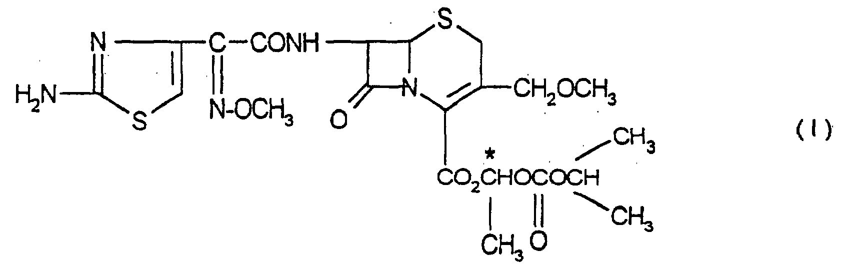 und deren physiologisch unbedenkliche Salze, worin die Verbindung der Formel I das polare Diastereomer ist.

2. Diastereomerenreine Salze der Verbindungen der allgemeinen Formel II 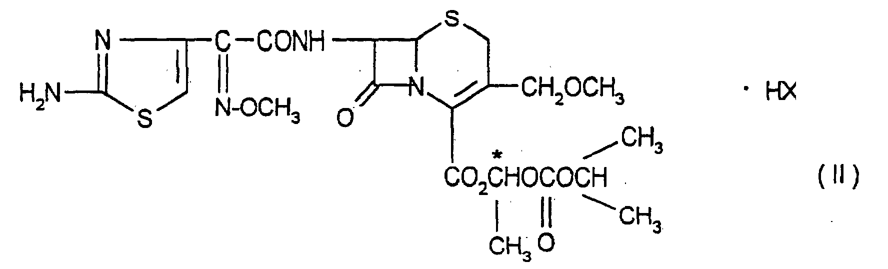 worin die Verbindung der Formel I das polarere Diastereomer ist und wobei HX für eine ein- oder mehrbasische Säure steht und wobei X für ein anorganisches oder organisches physiolgosich unbedenkliches Anion steht.

3. Diastereomerenreine Salze der Verbindungen der Formel II gemäß Anspruch 2, **dadurch gekennzeichnet, daß** HX für HCl, HBr und H₂SO₄ sowie für Methansulfonsäure, Ethansulfonsäure, Benzolsulfonsäure, p-Toluolsulfonsäure und 4-Ethylbenzolsulfonsäure steht.

4. Verfahren zur Herstellung von diastereomerenreinen Verbindungen der Formel I oder II gemäß den Ansprüchen 1 bis 3, **dadurch gekennzeichnet, daß** eine Zwischenstufe der Formel III 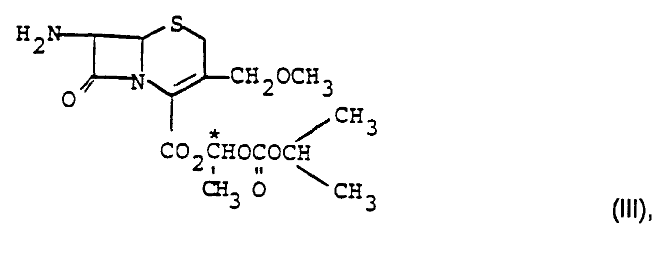 oder der Formel IV 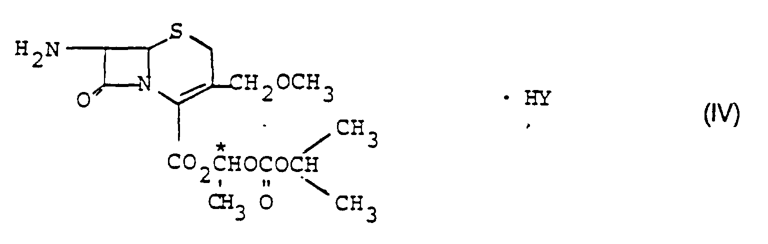 worin HY für eine ein- oder mehrbasische Säure, wobei Y ein anorganisches oder organisches Anion steht,
in diastereomerenreiner Form hergestellt und in die reinen Diastereomeren der Formel I oder der Formel 11 überführt wird.

5. Verfahren zur Herstellung von diastereomerenreinen Verbindungen der Formel I oder II gemäß Anspruch 4, **dadurch gekennzeichnet, daß** beim zusammengeben von 1 Äquivalent einer Lösung des Diastereomerengemisches der Formel III mit 0.2 - 2 Äquivalenten einer Lösung der Säurekomponente HY zunächst das schwerer lösliche Diastereomere der allgemeinen Formel IV gefällt und durch Filtration abgetrennt wird, anschließend aus der Filtrationslösung das leichter lösliche Diastereomere der allgemeinen Formel IV gefällt wird, wobei bei den aufeinanderfolgenden Teilschritten die Säurekomponente HY gleich oder verschieden sein kann und die Reihenfolge der Zugabe unterschiedlicher Säurekomponenten HY beliebig ist, und gegebenenfalls die erhaltenen Salze durch Kristallisation weiter gereinigt werden.

6. Gegen bakterielle Infektionen wirksame pharmazeutische Zubereitungen, **gekennzeichnet durch** einen wirksamen Gehalt einer diastereomerenreiner Verbindung der Formeln I oder II gemäß den Ansprüchen 1 bis 3.

7. Verfahren zur herstellung von gegen bakterielle Infektionen wirksamen pharmazeutischen Zubereitungen, **dadurch gekennzeichnet, daß** eine diastereomerenreine Verbindung der Formeln I oder II gemäß den Ansprüchen 1 bis 3 mit pharmazeutische üblichen Trägerstoffen und/oder Verdünnungsmitteln in eine pharmazeutisch geeignete Verabreichungsform gebracht wird.

8. Verwendung von diastereomerenreinen Verbindungen der Formeln I oder 11 gemäß den Ansprüchen 1 bis 3 zur Herstellung von Medikamenten mit antibakterieller Wirkung.

## Patentansprüche (Patentansprüche für folgende(n) Vertragsstaat(en): ES, GR)

1. Verfahren zur Herstellung von Diastereomeren des (6R,7R)-7-[2-(2-Aminothiazol-4-yl)-2-(Z)-(methoxyimino)acetamido]-3-(methoxymethyl)-3-cephem-4-carbonsäure-1-(isopropoxycarbonyloxy)ethylesters der Formel I 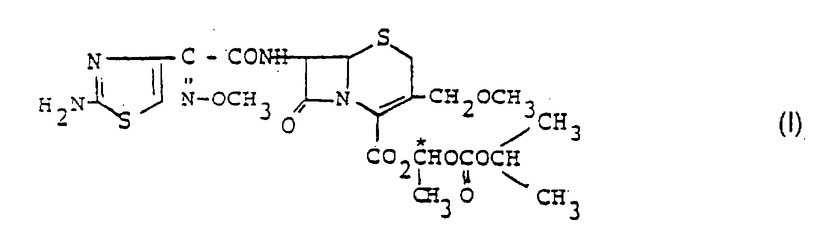 und diastereomerenreiner Salze der Verbindungen der allgemeinen Formel II 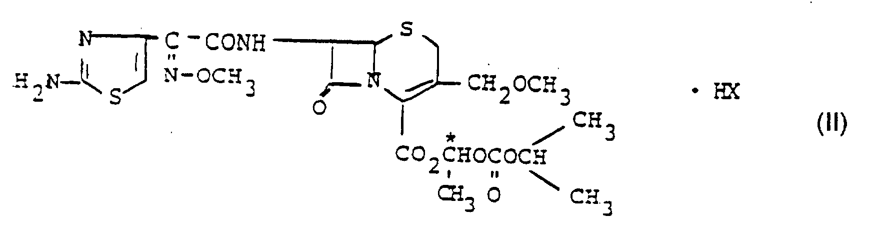 worin die Verbindung der Formel I oder der Formel II jeweils das polare Diastereomer ist, und wobei HX für eine ein- oder mehrbasische Säure steht und wobei X für ein anorganisches oder organisches physiologisch unbedenkliches Anion steht, **dadurch gekennzeichnet, daß** eine Zwischenstufe der Formel III 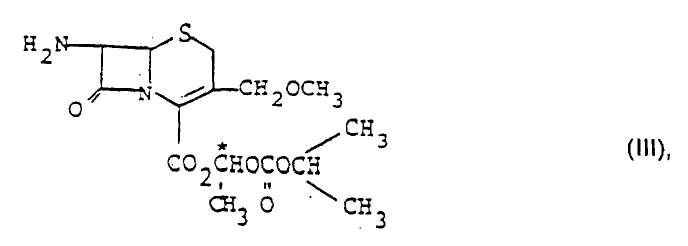 oder der Formel IV 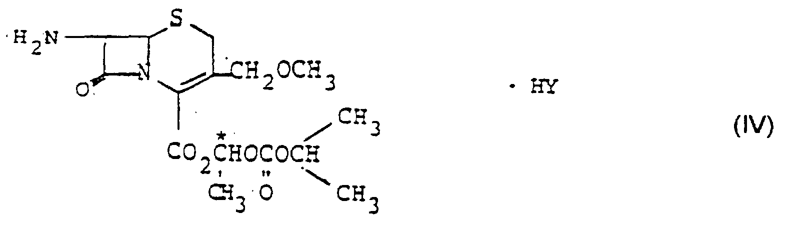 worin HY für eine ein- oder mehrbasische Säure, wobei Y für ein anorganisches oder organisches Anion steht,
in diastereomerenreiner Form hergestellt und in das reine Diastereomer der Formel I oder der Formel II überführt wird.

2. Verfahren zur Herstellung von diastereomerenreinen Verbindungen der Formel I oder II gemäß Anspruch 1, **dadurch gekennzeichnet, daß** beim zusammengeben von 1 Äquivalent einer Lösung des Diastereomerengemisches der Formel III mit 0.2 - 2 Äquivalenten einer Lösung der Säurekomponente HY zunächst das schwerer lösliche Diastereomere der allgemeinen Formel IV gefällt und durch Filtration abgetrennt wird, anschließend aus der Filtrationslösung das leichter lösliche Diastereomere der allgemeinen Formel IV gefällt wird, wobei bei den aufeinanderfolgenden Teilschritten die Säurekomponente HY gleich oder verschieden sein kann und die Reihenfolge der Zugabe unterschiedlicher Säurekomponenten HY beliebig ist, und gegebenenfalls die erhaltenen Salze durch Kristallisation weiter gereinigt werden.

3. Verfahren zur Herstellung von diastereomerenreinen Verbindungen der Formeln I oder II gemäß den Ansprüchen 1 bis 2, **dadurch gekennzeichnet, daß** HX für HCl, HBr und H₂SO₄ sowie für Methansulfonsäure, Ethansulfonsäure, Benzolsulfonsäure, p-Toluolsulfonsäure und 4-Ethylbenzolsulfonsäure steht.

4. Verfahren zur Herstellung von gegen bakterielle Infektionen wirksamen pharmazeutischen Zubereitungen mit einem wirksamen Gehalt einer diastereomerenreinen Verbindung der Formeln I oder II gemäß den Ansprüchen 1 bis 3, **dadurch gekennzeichnet, daß** eine diastereomerenreine Verbindung der Formeln I oder II mit pharmazeutische üblichen Trägerstoffen und/oder Verdünnungsmitteln in eine pharmazeutisch geeignete Verabreichungsform gebracht wird.

## Claims (Claims for the following Contracting State(s): AT, BE, CH, DE, DK, FR, GB, IE, IT, LI, LU, NL, PT, SE)

1. A diastereomer of 1-(isopropoxycarbonyloxy)ethyl (6R,7R)-7-[2-(2-aminothiazol-4-yl)-2-(Z)-(methoxyimino)acetamido)-3-(methoxymethyl)-3-cephem-4-carboxylate of the formula I 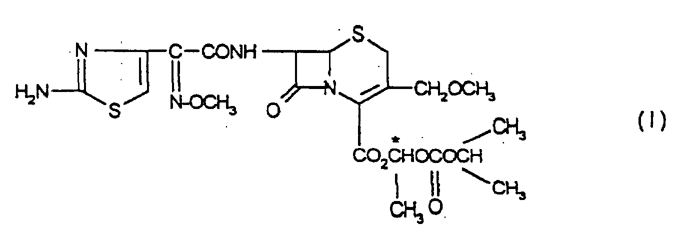 or its physiologically acceptable salts in which the compound of the formula I is the polar diastereomer.

2. A diastereomerically pure salt of the compound of the formula II 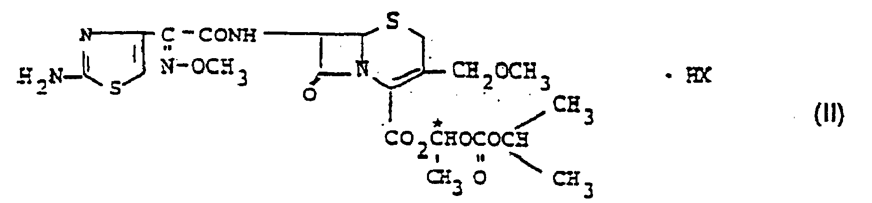 in which the compound of the formula I is the polar diastereomer and where HX is a mono- or polybasic acid and where X is an inorganic or organic physiologically acceptable anion.

3. A diastereomerically pure salt of the compound of the formula II as claimed in claim 2, wherein HX is HCl, HBr or H₂SO₄ or methanesulfonic acid, ethanesulfonic acid, benzenesulfonic acid, p-toluenesulfonic acid or 4-ethylbenzenesulfonic acid.

4. A process for the preparation of diastereomerically pure compounds of the formula I or II as claimed in any of claims 1 to 3, which comprises preparing an intermediate of the formula III 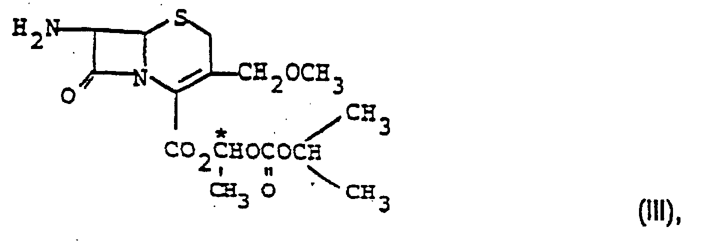 or of the formula IV 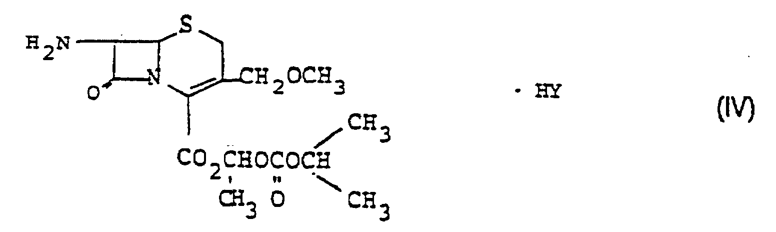 in which HY is a mono- or polybasic acid, where Y is an inorganic or organic anion,
in diastereomerically pure form and converting it into the pure diastereomers of the formula I or of the formula II.

5. The process for the preparation of diastereomerically pure compounds of the formula I or II as claimed in claim 4, wherein in the mixing together of 1 equivalent of a solution of the diastereomer mixture of the formula III with 0.2-2 equivalents of a solution of the acid component HY the more sparingly soluble diastereomer of the formula IV is first precipitated and separated off by filtration, then the more readily soluble diastereomer of the formula IV is precipitated from the filtration solution, it being possible in the subsequent partial steps for the acid component HY to be identical or different and any desired sequence of addition of different acid components HY being possible, and the obtained salts optionally being further purified by crystallization.

6. A pharmaceutical preparation which is active against bacterial infections, which comprises an effective content of a diastereomerically pure compound of the formula I or II as claimed in any of claims 1 to 3.

7. A process for the production of pharmaceutical preparations which are effective against bacterial infections, which comprises bringing a diastereomerically pure compound of the formula I or II as claimed in any of claims 1 to 3 into a pharmaceutically suitable administration form using pharmaceutically customary excipients and/or diluents.

8. The use of diastereomerically pure compounds of the formula I or II as claimed in any of claims 1 to 3 for the manufacture of drugs having an antibacterial effect.

## Claims (Claims for the following Contracting State(s): ES, GR)

1. A process for the preparation of diastereomers of 1-(isopropoxycarbonyloxy)ethyl (6R,7R)-7-[2-(2-aminothiazol-4-yl)-2-(Z)-(methoxyimino)acetamido]-3-(methoxymethyl)-3-cephem-4-carboxylate of the formula I 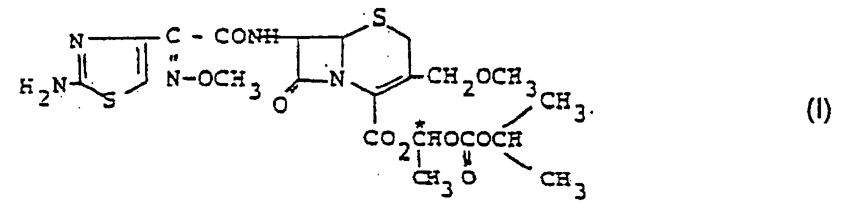 and diastereomerically pure salts of the compounds of the formula II 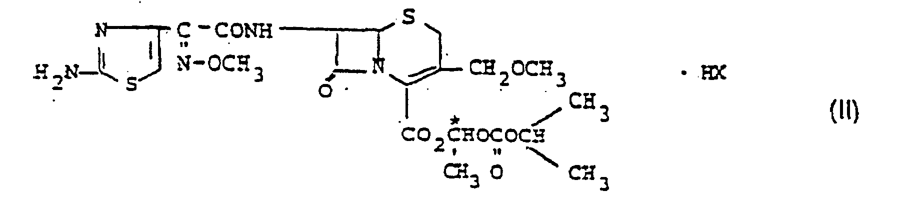 in which the compound of the formula I or of the formula II is respectively the polar diastereomer, and where HX is a mono- or polybasic acid and where X is an inorganic or organic physiologically acceptable anion, which comprises preparing an intermediate of the formula III 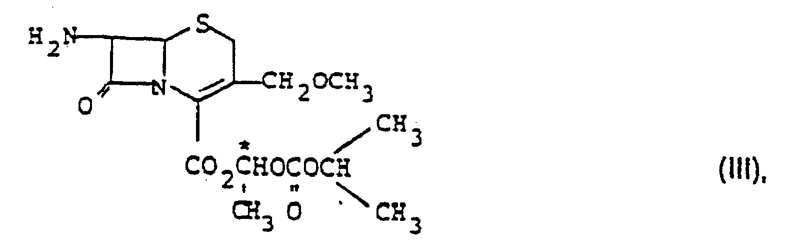 or of the formula IV 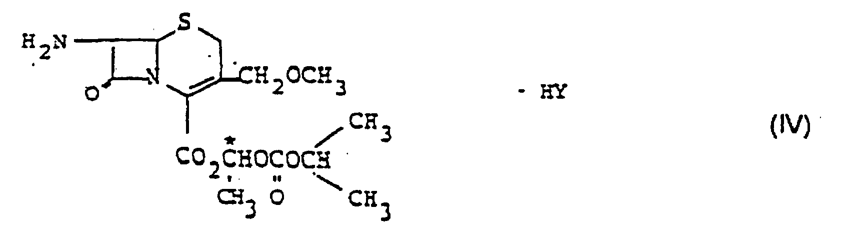 in which HY is a mono- or polybasic acid, where Y is an inorganic or organic anion,
in diastereomerically pure form and converting it into the pure diastereomer of the formula I or of the formula II.

2. The process for the preparation of diastereomerically pure compounds of the formula I or II as claimed in claim 1, wherein in the mixing together of 1 equivalent of a solution of the diastereomer mixture of the formula III with 0.2-2 equivalents of a solution of the acid component HY the more sparingly soluble diastereomer of the formula IV is first precipitated and separated off by filtration, then the more readily soluble diastereomer of the formula IV is precipitated from the filtration solution, it being possible in the subsequent partial steps for the acid component HY to be identical or different and any desired sequence of addition of different acid components HY being possible, and the obtained salts optionally being further purified by crystallization.

3. The process for the preparation of diastereomerically pure compounds of the formula I or II as claimed in claims 1 and 2, wherein HX is HCl, HBr or H₂SO₄ or methanesulfonic acid, ethanesulfonic acid, benzenesulfonic acid, p-toluenesulfonic acid or 4-ethylbenzenesulfonic acid.

4. A process for the production of pharmaceutical preparations having an effective content of a diastereomerically pure compound of the formula I or II as claimed in any of claims 1 to 3 and which are effective against bacterial infections, which comprises bringing a diastereomerically pure compound of the formula I or II into a pharmaceutically suitable administration form using pharmaceutically customary excipients and/or diluents.

## Revendications (Revendications pour l'(les) Etat(s) contractant(s) suivant(s): AT, BE, CH, DE, DK, FR, GB, IE, IT, LI, LU, NL, PT, SE)

1. Diastéréomères du composé ester 1-(isopropoxycarbonyloxy)éthylique d'acide (6R,7R)-7-[2-(2-aminothiazol-4-yl)-2-(Z)-(méthoxyimino)acétamido]-3-(méthoxyméthyl)-3-céphèm-4-carboxylique de formule I 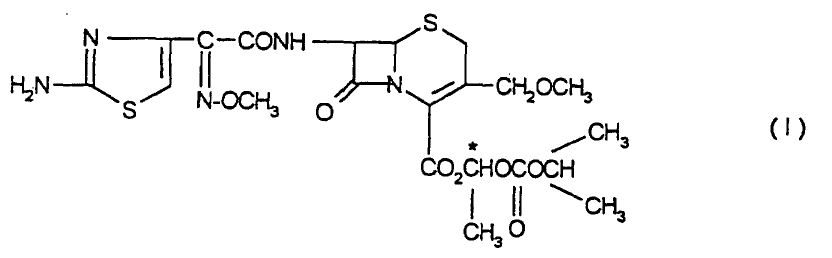 et de ses sels physiologiquement acceptables, dans laquelle le composé de formule I est le diastéréomère polaire.

2. Sels de diastéréomères purs des composés de formule générale II 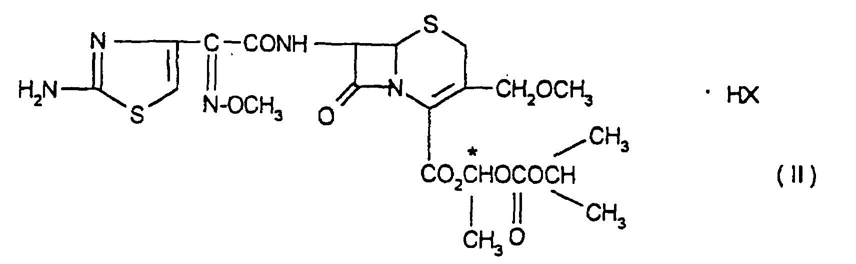 dans laquelle le composé de formule I est le diastéréomère polaire et dans laquelle HX représente un acide mono ou polybasique et dans laquelle X représente un anion inorganique ou organique physiologiquement acceptable.

3. Sels de diastéréomères purs des composés de formule II selon la revendication 2, **caractérisés en ce que** HX représente HCl, HBr et H₂SO₄ ainsi que l'acide méthanesulfonique, l'acide éthanesulfonique, l'acide benzènesulfonique, l'acide p-toluènesulfonique et l'acide 4-éthylbenzènesulfonique.

4. Procédé pour la préparation de composés diastéréomères purs de formule I ou II selon les revendications 1 à 3, **caractérisé en ce qu'**on prépare un produit intermédiaire de formule III 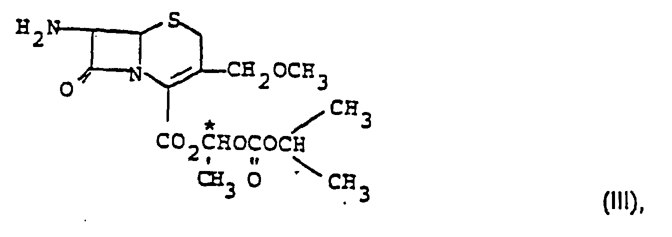 ou de formule IV 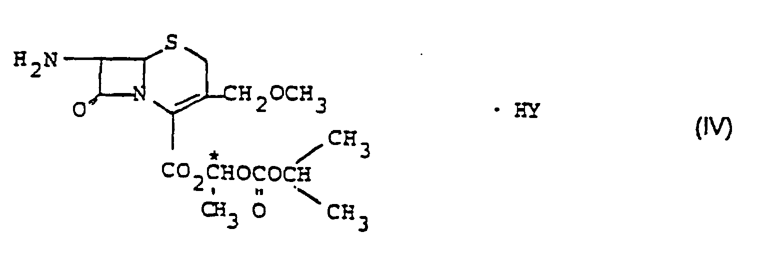 dans laquelle HY représente un acide mono ou polybasique, dans laquelle Y représente un anion inorganique ou organique,
sous forme diastéréomère pure et **en ce qu'**on le transforme en les diastéréomères de formule I ou de formule II.

5. Procédé pour la préparation de composés diastéréomères purs de formule I ou II selon la revendication 4, **caractérisé en ce qu'**on fait d'abord précipiter le diastéréomère difficilement soluble de formule générale IV par ajout à 1 équivalent d'une solution du mélange des diastéréomères de formule III de 0,2-2 équivalents d'une solution du composant acide HY et **en ce qu'**on le sépare par filtration, on précipite ensuite de la solution de filtration le diastéréoisomère faiblement soluble de formule générale IV, dans lequel dans les étapes partielles successives le composant acide HY peut être identique ou différent et l'ordre de l'addition des différents composants acides HY est quelconque, et on continue éventuellement à purifier les sels obtenus par cristallisation.

6. Préparations pharmaceutiques actives contre les infections bactériennes, **caractérisées par** une concentration efficace d'un composé diastéréomère pur des formules I ou II selon les revendications 1 à 3.

7. Procédé de fabrication de préparations pharmaceutiques actives contre les infections bactériennes, **caractérisé en ce qu'**on met un composé diastéréomère pur des formules I ou II selon les revendications 1 à 3 avec des substances support usuelles et/ou des agents de dilution sous une forme d'administration pharmaceutiquement appropriée.

8. Utilisation de composés diastéréomères purs des formules I ou II selon les revendications 1 à 3 pour la préparation de médicaments avec une activité antibactérienne.

## Revendications (Revendications pour l'(les) Etat(s) contractant(s) suivant(s): ES, GR)

1. Procédé pour la préparation de diastéréomères de l'ester 1-(isopropoxycarbonyloxy)éthylique d'acide (6R,7R)-7-[2-(2-amino-thiazol-4-yl)-2-(Z)-(méthoxyimino)-acétamido]-3-(méthoxyméthyl)-3-céphèm-4-carboxylique de formule I 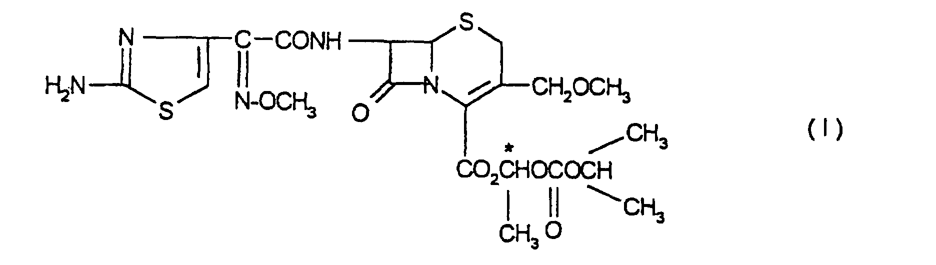 et des sels diastéréomères purs des composés de formule générale II 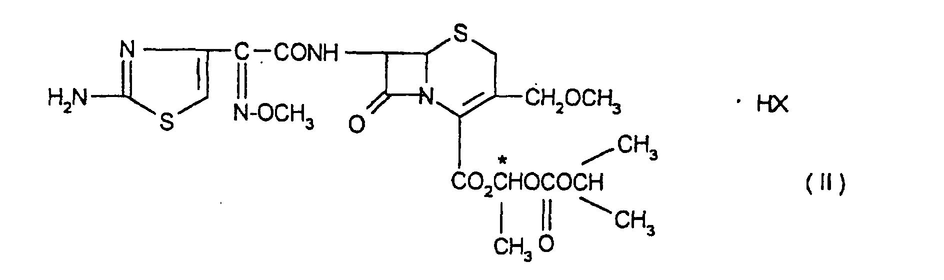 dans lequel le composé de formule I ou de formule II est respectivement le diastéréomère polaire, et dans lequel HX représente un acide mono ou polybasique et dans lequel X représente un anion inorganique ou organique physiologiquement acceptable, **caractérisé en ce qu'**on prépare un produit intermédiaire de formule III 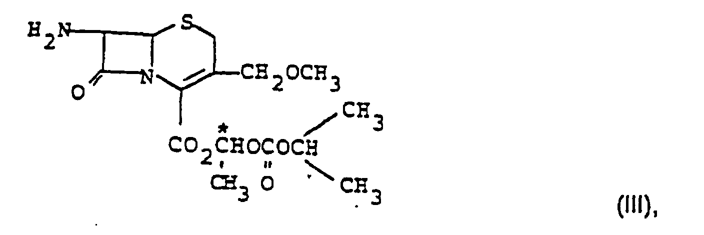 ou de formule IV 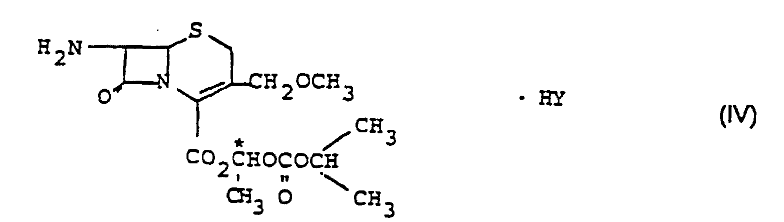 dans laquelle HY représente un acide mono ou polybasique, dans laquelle Y représente un anion inorganique ou organique,
sous forme diastéréomère pure et **en ce qu'**on le transforme en le diastéréomère pur de formule I ou de formule II.

2. Procédé pour la préparation de composés diastéréomères purs de formule I ou II selon la revendication 1, **caractérisé en ce qu'**on fait d'abord précipiter le diastéréomère difficilement soluble de formule générale IV par ajout à 1 équivalent d'une solution du mélange des diastéréomères de formule III de 0,2-2 équivalents d'une solution du composant acide HY et **en ce qu'**on le sépare par filtration, on précipite ensuite de la solution de filtration le diastéréoisomère faiblement soluble de formule générale IV, dans lequel dans les étapes partielles successives le composant acide HY peut être identique ou différent et l'ordre de l'addition des différents composants acides HY est quelconque, et on continue éventuellement à purifier les sels obtenus par cristallisation.

3. Procédé pour la préparation de composés diastéréomères des formules I ou II selon les revendications 1 et 2, **caractérisé en ce que** HX représente HCl, HBr et H₂SO₄ ainsi que l'acide méthanesulfonique, l'acide éthanesulfonique, l'acide benzènesulfonique, l'acide p-toluènesulfonique et l'acide 4-éthylbenzènesulfonique.

4. Procédé de fabrication de préparations pharmaceutiques actives contre les infections bactériennes avec une teneur efficace d'un composé diastéréomère des formules I ou II selon les revendications 1 à 3, **caractérisé en ce qu'**on met un composé diastéréomère pur des formules I ou II sous une forme d'administration pharmaceutiquement appropriée avec des substances support usuelles et/ou des agents de dilution.
